(19) Europäisches Patentamt / European Patent Office / Office européen des brevets

(11) **EP 3 939 701 A1**

(12) **EUROPEAN PATENT APPLICATION**
published in accordance with Art. 153(4) EPC

(43) Date of publication:
**19.01.2022 Bulletin 2022/03**

(21) Application number: **20770856.1**

(22) Date of filing: **11.03.2020**

(51) International Patent Classification (IPC):
***B01L 3/00*** *(2006.01)*

(52) Cooperative Patent Classification (CPC):
**B01L 3/00**

(86) International application number:
**PCT/KR2020/003411**

(87) International publication number:
**WO 2020/184992 (17.09.2020 Gazette 2020/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2019 KR 20190028069**
           **25.10.2019 KR 20190134094**
           **25.10.2019 KR 20190134095**

(71) Applicant: **Korea University Research and Business Foundation**
**Seoul 02841 (KR)**

(72) Inventors:
• **CHUNG, A-Ram**
 **Seoul 04425 (KR)**
• **KANG, Gum-Young**
 **Jinju-si, Gyeongsangnam-do 52702 (KR)**
• **HUR, Jeong-Soo**
 **Seoul 06289 (KR)**

(74) Representative: **Meissner Bolte Partnerschaft mbB**
**Patentanwälte Rechtsanwälte**
**Postfach 86 06 24**
**81633 München (DE)**

(54) **MICROFLUIDIC SYSTEM FOR INTRACELLULAR DELIVERY OF SUBSTANCES AND METHOD THEREFOR**

(57) There is provided a microfluidic system delivering external materials into a cell by cell mechanoporation using inertia, the microfluidic system including a fluidic channel structure through which a solution containing a cell and external materials flows continuously, in which the fluidic channel structure includes a junction between one or more channels, a localized vortex is generated near an interface of the junction, the cell is deformed by the vortex, and transient discontinuities are generated in a cell membrane by the vortex and the external materials are introduced into the cell by solution exchange between the cell and fluid around the cell.

[FIG. 1]

A Spiral Hydroporator

## Description

## TECHNICAL FIELD

The present disclosure relates to a microfluidic system for intracellular delivery of materials and a method therefor.

## BACKGROUND ART

Intracellular delivery of materials is one of the most key steps in cell engineering, in which materials are traditionally delivered by using a carrier or by physically making nanopores in a cell/nuclear membrane. For virus or lipid carrier techniques, it is possible to deliver materials with high efficiency when optimized, but there are drawbacks such as low safety, slow delivery speed, labor/cost-intensive carrier preparation process, and low reproducibility.

On the other hand, for methods of physically making a nanopore by applying energy to a cell membrane (e.g., electroporation or microneedle), there is an advantage that relatively various materials are able to be delivered to various cell lines. However, low cell viability, denaturation of delivery material, and low throughput, which are caused by the invasiveness of the methods, are pointed out as major limitations.

To address the above-mentioned drawbacks, microfluidic devices capable of processing a large number of cells are prominently used. Typically, there is a platform that creates nanopores in cell membranes through physical deformation of cells when the cells pass through the bottleneck section. However, the approach has major drawbacks such as clogging of the bottleneck section itself during the experiment and inconsistent material delivery efficiency.

For example, US Patent No. 2014-0287509 discloses a technology for inducing cell deformation by applying pressure to cells through a channel having a bottleneck structure. However, in this case, there is a drawback that the cells block the bottleneck structure, and there is a drawback that it is possible to deliver materials only to cells smaller than the size of a constriction. Furthermore, there is also a drawback that the cost rises because a channel having a fine diameter has to be used.

Therefore, it is urgent to develop an innovative next-generation intracellular material delivery platform capable of delivering various materials into cells uniformly and with high efficiency while making use of the high processing function of the microfluidic device.

## DISCLOSURE OF THE INVENTION

## TECHNICAL PROBLEM

Accordingly, in order to solve the above-mentioned problems, an object of the present disclosure is to provide a system and method capable of delivering materials to a large number of cells with high efficiency without using a new active delivery means.

## TECHNICAL SOLUTION

In order to solve the problems described above, according to an aspect of the present disclosure, there is provided a microfluidic system delivering external materials into a cell by cell mechanoporation using inertia and inertial effects, the microfluidic system including a fluidic channel structure through which a solution containing a cell and external materials flows continuously, in which the fluidic channel structure includes a junction between one or more channels, a localized vortex is generated near an interface of the junction, the cell is deformed by the vortex, and transient discontinuities are generated in a cell membrane by fluidic cell deformation and the external materials are introduced into the cell via solution exchange between the cell and fluid around the cell.

In an embodiment of the present disclosure, the fluidic channel structure including the junction between one or more channels may include a junction including a T, Y, cross shape, or a combination thereof.

In an embodiment of the present disclosure, the fluidic channel structure may include a cavity near a fluid stagnation point when the fluidic channel structure is a channel of the T or Y shape.

In an embodiment of the present disclosure, the cavity may have a shape of a circle, an ellipse, an elongate slit, a square, a rectangle, a trapezoid, a polygon, and a combination thereof, and a modification thereof.

In an embodiment of the present disclosure, a diameter of the cavity may be determined according to a diameter of the cell.

In an embodiment of the present disclosure, the microfluidic system may further include fluid control unit for allowing a solution to flow in the fluidic channel structure, and the fluid control unit may allow the solution to flow in the fluidic channel at a velocity that is at a level capable of generating a localized vortex near the interface of the junction.

In an embodiment of the present disclosure, the fluid control unit may be a syringe pump or pneumatic system.

In an embodiment of the present disclosure, a Reynolds number (Re) of the solution may be 1 to 1000.

In an embodiment of the present disclosure, the vortex feature may be determined by the Reynolds number.

In an embodiment of the present disclosure, the vortex may be in a form of a closed or open recirculating flow.

In an embodiment of the present disclosure, the microfluidic system may be formed by combining the microfluidic system according to any one of claims 1 to 12 in series, parallel, or a combination thereof.

According to another aspect of the present disclosure, there is provided a method of delivering external materials into a cell by cell mechanoporation using inertia and inertial effects, the method including: allowing a solution containing the cell and external materials to continuously flow a fluidic channel; forming a vortex by vortex generating means near the junction; deforming the cell by the vortex; and allowing the external materials to be introduced into the cell through a pore created in a cell membrane by the deforming of the cell.

in an embodiment of the present disclosure, the vortex generating means may be a junction structure of the fluidic channels.

In an embodiment of the present disclosure, the fluidic channel may include a junction including a T, Y, cross shape, or a combination thereof.

## ADVANTAGEOUS EFFECTS

According to the present disclosure, a vortex is generated by allowing a cell and external materials to flow into a fluidic channel structure including at least one junction, and the resulting inertia and inertial effects deform the cell to induce transient discontinuity in a cell membrane, thereby perforating the cell membrane. Then, a solution exchanges between the cell and fluid around the cell occurs through the perforated cell membrane, and as a result, the external materials are introduced into the cell. Thus, the present disclosure does not require vectors or active cell delivery means (e.g., electric fields). Therefore, the present disclosure may directly deliver external materials (e.g., genes, plasmids, nanoparticles, or the like) into cells with high efficiency and low cost only by solution and channel structure features.

## BRIEF DESCRIPTION OF THE DRAWINGS

FIG. 1 is a diagram for illustrating a formation of a spiral vortex in a + junction channel.
FIG. 2 is a schematic diagram of cell deformation caused by a spiral vortex (1) and shows high-speed microscopy images showing rotational cell motion (scale bar: 10 $\mu$m)(2).
FIG. 3 is a diagram for describing a mechanism of material delivery with cell deformation according to an embodiment of the present disclosure.
FIG. 4 is a schematic diagram of two types of channel structures according to an embodiment of the present disclosure and a diagram for describing cell deformation in each channel.
FIG. 5 is a schematic diagram of a microfluidic system in which two types of fluidic channels of FIG. 4 are connected in series in a fluid flow direction.
FIG. 6 shows a result of an experiment on behavior according to a Reynolds number of fluid flowing in opposite directions toward a junction (intersection) in a + junction channel.
FIG. 7 shows high-speed microscopy images showing a cell (MDA-MB-231) trapping behavior in the + junction channel.
FIG. 8 shows a graph of normalizing cell trapping and cell deformation time by vortex breakdown of FIG. 7 as a function of the Reynolds number.
FIG. 9 is a diagram for illustrating an intracellular material delivery platform according to an embodiment of the present disclosure.
FIGS. 10 to 12 are diagrams for illustrating a method for intracellular delivery of materials using the intracellular material delivery platform according to an embodiment of the present disclosure.
FIG. 13 is a schematic diagram of a T-junction channel according to an embodiment of the present disclosure.
FIG. 14 shows high-speed microscopy images illustrating a cell deformation and vortex deformation mechanism of a T-junction channel of a cavity structure. Here, each arrow indicates a fluid flow direction.
FIG. 15 shows a vortex deformability index (VDI) analysis result in a fluid having different Reynolds numbers.
FIG. 16 is a diagram for illustrating an intracellular material delivery platform according to an embodiment of the present disclosure.
FIGS. 17 to 19 are diagrams for illustrating a method for intracellular delivery of materials using the intracellular material delivery platform according to an embodiment of the present disclosure.
FIGS. 20 and 21 show analysis results of the amount of materials delivered into cells.
FIG. 22 shows a result of measuring cell viability by varying the Reynolds number under the same conditions as in FIG. 19.
FIG. 23 shows an analysis result of delivery efficiency for various dextran sizes.
FIG. 24 shows a result of measuring dextran delivery efficiency in the complex microfluidic system of FIG. 5 (a combination of the + junction channel and the T-junction channel) .
FIG. 25 shows a comparison result of the delivery efficiency of electroporation in the related art (a neon transfection system (Thermo Fisher Scientific, Waltham, MA, USA)) and the method for delivery of materials based on inertia (hydroporation) according to the present disclosure.
FIGS. 26 and 27 are photographs for analyzing the intracellular delivery effect of gold nanoparticles (GNPs), and a result of counting scattering spots, respectively.
FIG. 28 shows a result of measuring cell viability depending on GNP delivery.
FIG. 29 shows a result of calculating a yield normalized by multiplying the number of scattering spots by the cell viability.
FIG. 30 shows an analysis result of intracellular delivery efficiency of mesooporous silica nanoparticles (MSN) used as a drug, protein, and nucleic acid nanocarrier instead of gold nanoparticles.

FIG. 31 shows an analysis result of a time-dependent cell viability for the analyte of FIG. 30.

FIG. 32 is a histogram of fluorescence intensity obtained by measuring a delivery effect to K562 cells using mRNA (996-nucleotide mRNA fragment, green fluorescent protein) as an external material.

FIG. 33 shows fluorescence images after delivery of mRNA into K562 by (C) endocytosis and (D) the microfluidic system according to the present disclosure.

FIG. 34 shows a result of measuring delivery efficiency (E) and viability (F) when mRNA is delivered according to the present disclosure as shown in FIGS. 32 and 33.

FIG. 35 shows fluorescence images after mRNA (996-nucleotide mRNA fragment, green fluorescent protein) is delivered into K562 cells by endocytosis and a method of Embodiment 2.

FIG. 36 shows an analysis result of (b) mRNA transfection efficiency and (c) mean fluorescence intensity depending on mRNA concentration.

FIG. 37 shows fluorescence images after delivery of plasmid DNA into HEK293t by the endocytosis (control) and the method of Embodiment 2.

FIG. 38 shows a graph of (e) analyzing plasmid DNA (pDNA) transfection efficiency of HEK293t cells and (f) mean intensity of HEK293t cells depending on plasmid DNA concentration.

FIGS. 39 and 40 are a result of western blot analysis of the ITGa1 gene knockdown effect of HeLa cells ($\alpha$1 subunit of an integrin transmembrane receptor) into which siRNA is delivered, and a result of comparing relative expression levels, respectively.

FIG. 41 shows an analysis result of transfection yields by Lipofectamine 3000, electroporation, and the present disclosure.

FIGS. 42 to 44 are analysis results of transfection efficiencies, cell viability, and transfection yields for human MSC, human ADSC, and mouse BMDC.

FIG. 45 shows confocal microscopy images showing delivery of quantum dots (qdot625) into MDA-MB-231 cells by the present disclosure ($\mu$-Hydroporator), electroporator, and Lipofectamine 3000.

FIG. 46 shows an analysis result of spot number counts of quantum dots (Qdot 625) per cell.

FIG. 47 is a fluorescence intensity histogram of Embodiment ($\mu$-Hydroporator, $N_{cell}$ = 5,000 per sample) in which nanospheres (green fluorescent silica nanospheres, Micromod, Germany) are delivered into K562 cells, by a negative control (nontreated K562 cells), a positive control (K562 cells co-incubated with nanospheres, the same time and concentration as those in Embodiment), and the present disclosure ($\mu$-Hydroporator).

FIG. 48 shows a result of measuring relative mean fluorescence intensity.

FIGS. 49 and 50 show confocal images of two of the groups of FIGS. 47 and 48.

## BEST MODE FOR CARRYING OUT THE INVENTION

Hereinafter, preferred embodiments of a system for intracellular delivery of materials based on inertia according to the present disclosure will be described in detail with reference to the accompanying drawings. For reference, it should be understood that the terms used in the specification and the appended claims should not be construed as limited to general and dictionary meanings, but should be interpreted based on the meanings and concepts corresponding to technical aspects of the present disclosure on the basis of the principle that the inventor is allowed to define terms appropriately for the best explanation. In addition, the embodiment disclosed in the present disclosure and configurations shown in the accompanying drawings are just one preferred embodiment of the present disclosure and do not represent all technical ideas of the present disclosure. Therefore, it should be understood that the present disclosure covers various modifications and variations provided they come within the scope of the appended claims and their equivalents at the time of filing of this application.

In order to solve the problems described above, the present disclosure provides a microfluidic system delivering external materials into a cell by cell mechanoporation using inertia and inertial effects, the microfluidic system including a fluidic channel structure through which a solution containing a cell and external materials flows continuously, in which the fluidic channel structure includes a junction between one or more channels, a localized vortex is generated near an interface of the junction, the cell is deformed by the vortex, and a transient discontinuous shape of a cell membrane is generated by the vortex and the external materials are introduced into the cell by solution exchange between the cell and fluid around the cell.

In addition, the present disclosure provides a method of delivering external materials into a cell by cell mechanoporation using inertia and inertial effects, the method including: allowing a solution containing the cell and external materials to continuously flow a fluidic channel; forming a vortex by a vortex generating means near the junction; deforming the cell by the vortex; and allowing the external materials to be introduced into the cell through a pore created in a cell membrane by the deforming of the cell.

## MODE FOR CARRYING OUT THE INVENTION

A method and system for intracellular delivery of materials according to an embodiment of the present disclosure are based on a technical feature of generating a vortex in a fluidic channel and deforming cells by the vortex to create a pore in cell membranes. In particular, the present disclosure improves the intracellular material delivery efficiency by cell deformation caused by the vortex and cell deformation by subsequent vortex breakdown, when cells flow into the vortex. In an embodiment of the present disclosure, the vortex is generated through a physical structure of a fluidic channel, such as a junction, but the scope of the present disclosure is not limited thereto.

According to an embodiment in which a vortex generating means is the junction, the present disclosure provides a microfluidic system having a fluidic channel structure including one or more junctions at which at least two channels are connected, as a system for delivering an external material outside a cell into the cell. In the present disclosure, "junction" means a point at which each channel meets another channel when two or more channels are connected in the form of T, Y, + or a combination thereof, and in the present disclosure, at least one junction may be provided in a single channel defined by an inlet and an outlet of a fluid.

In the microfluidic system according to an embodiment of the present disclosure, a vortex is generated at an interface of the junction as the solution containing cells and external materials flows into the junction, and at this time, cells that continuously experience the vortex and vortex breakdown are continuously trapped and deformed by inertia and inertial effects, and then pores in cell membranes, which are pathways for intracellular delivery of materials, are formed one after another as the deformation progresses. That is, the microfluidic system according to the present disclosure may greatly improve the intracellular material delivery efficiency by utilizing a fluid property such as a Reynolds number (1 to 1000) and the channel structure in the form of T, Y, + or a combination thereof.

In the microfluidic system of the following embodiments of the present disclosure, a mold with channels according to the present disclosure formed is first made by etching a SU-8 mold or a silicon wafer through a normal photolithography process. Then, a polydimethylsiloxane (PDMS)-based chip is made through PDMS, and at this time, an inlet and an outlet are made in the chip made as mentioned above, and general slide glass is combined using plasma treatment (Cute, Femto Science, South Korea), thereby making a platform device. Then, cells in the suspended state and materials to be delivered are mixed, and then the mixture is injected into the made chip by using a syringe pump. In this case, the intracellular delivery of materials may be controlled by adjusting the flow rate of the syringe pump, and after the delivery, only the cells are separated by using a centrifuge, and then cultured or analyzed or used according to the purpose. However, the scope of the present disclosure is not limited to the embodiments themselves.

Embodiment 1: + Junction Structure Microfluidic System

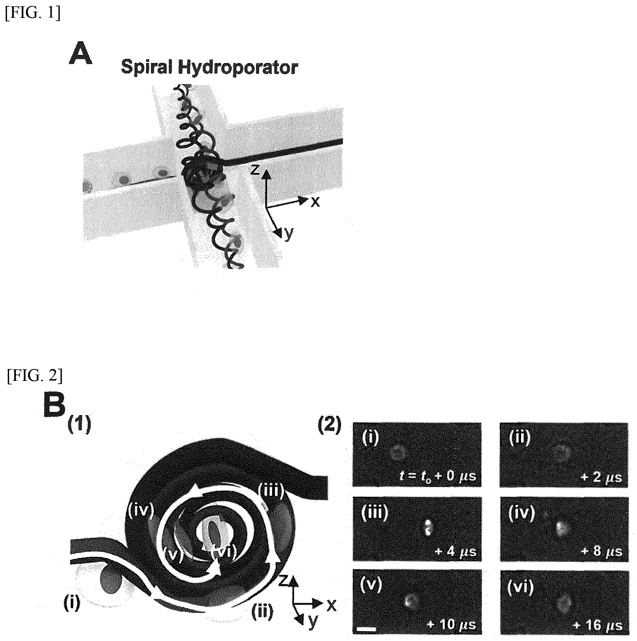

FIG. 1 is a diagram for illustrating a formation of a spiral vortex in a + junction channel.

Referring to FIG. 1, as fluid flows in opposite directions at both ends of the + channel, the fluid exits into two other channels intersecting the channel, and at this time, fluid instability near a stagnation point induces a strong spiral localized vortex near a proximal point. The local vortex explains the reasons for the phenomena of the cell deformation and intracellular delivery of materials described below. In the present disclosure, "near" refers to a region in which a vortex of a fluid flowing into a junction may be generated due to a junction structure.

FIG. 2 is (1) a schematic diagram of cell deformation caused by a spiral vortex and shows high-speed microscopy images (scale bar: 10 $\mu$m) showing rotational cell motion.

Referring to FIG. 2, in the present disclosure, when allowing fluid to flow to a junction channel with a medium Reynolds number (1 to 1000), cells move spirally instead of symmetrical cell elongation, and at this time, the cells show a large deformation as the cells approach a stagnation point of the vortex flow.

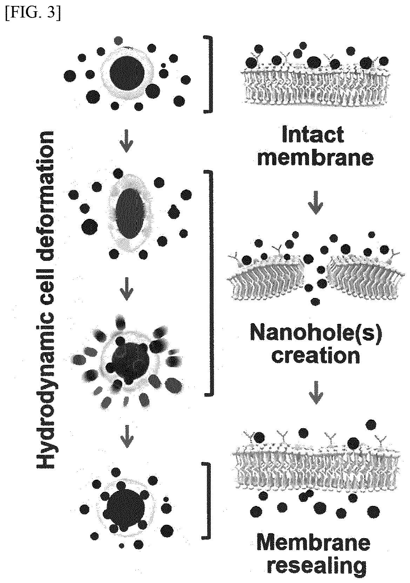

FIG. 3 is a diagram for describing a mechanism of material delivery with cell deformation according to an embodiment of the present disclosure.

Referring to FIG. 3, a nanopore is created in a cell membrane by the cell deformation on the left, and the external materials are introduced into the cell by solution exchange between the cell and fluid around the cell. Then, within 1 to 10 minutes, the cell membrane self-recovers and closes, and the recovery time may be controlled by adjusting the concentration of an electrolyte such as calcium in the solution.

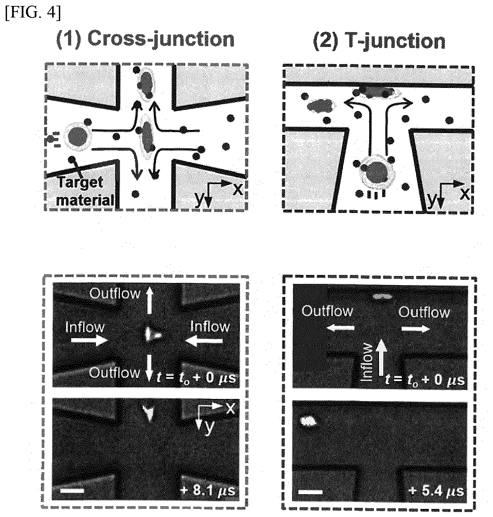

FIG. 4 is a schematic diagram of two types of channel structures according to an embodiment of the present disclosure and a diagram for describing cell deformation in each channel.

The two types of channel structures in FIG. 4 are a + structure in which oppositely flowing fluid collides near the junction to form a vortex near the junction, and a T-shaped structure in which the introduced cells collide with a channel wall.

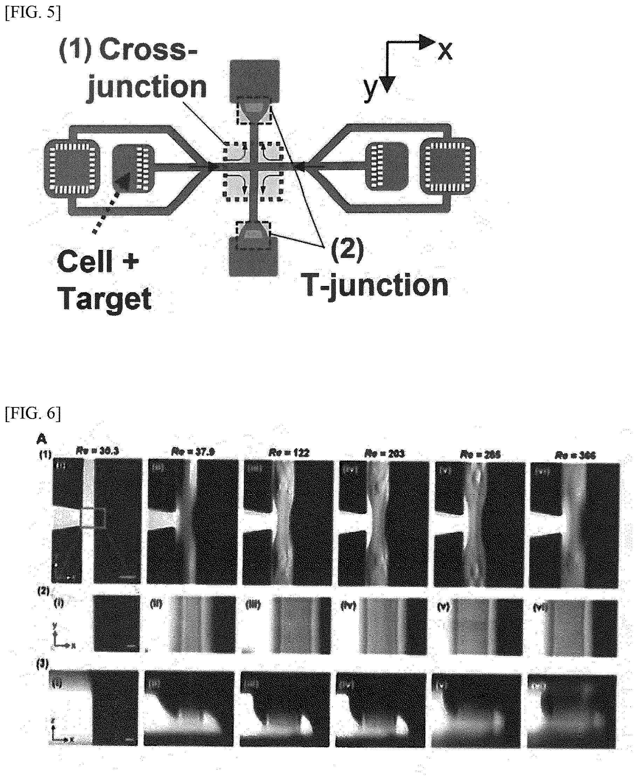

FIG. 5 is a schematic diagram of a microfluidic system in which two types of fluidic channels of FIG. 4 are connected in series in a fluid flow direction.

Referring to FIG. 5, the microfluidic system of the present disclosure has (1) a + junction channel (cross-junction) and (2) a T-junction channel (T-junction) in which a fluid guided while passing through the vortex from the + junction channel collides with the channel wall.

For the above-mentioned system, cells out of the stagnation point in the + junction channel (cross-junction) are again guided to a channel center by inertia and collide with the channel wall again. That is, the T, Y, and +-shaped microfluidic structures according to the present disclosure may be designed in series, parallel, or a combination thereof with respect to fluid flow, all of which fall within the scope of the present disclosure. In this case, a plurality of junctions may be formed that connects a plurality of unit channels on a single channel defined by an inlet and an outlet in terms of cells.

FIG. 6 shows a result of an experiment on behavior according to the Reynolds number of fluid flowing in opposite directions toward a junction (intersection) in the + junction channel.

Referring to Fig. 6, at the lowest Reynolds number, the interface between the two fluid streams remains clearly symmetrical, and the three-dimensional vortex motion clearly starts at the Reynolds number 37.9 (critical Reynolds number). Then, with the increase in the Reynolds number, the intersection between the two fluid streams becomes stronger and more complex (see (1)).

(2) and (3) in FIG. 6 are confocal microscopy images, where, with the increase in the Reynolds number, the counterclockwise spiral vortex develops beyond the critical Reynolds number, and the spiral expands vertically and horizontally. The above result means that the vortex is generated near the junction and its shape, pattern, velocity, and the like vary depending on the Reynolds number of the fluid. This also suggests that delivery efficiency may be improved by varying the Reynolds number depending on the desired cell type and the type of materials to be delivered.

The system according to the present disclosure has a specific additional effect of trapping cells and inducing deformation thereof by cell deformation due to vortex formation and vortex breakdown after the delivery of materials.

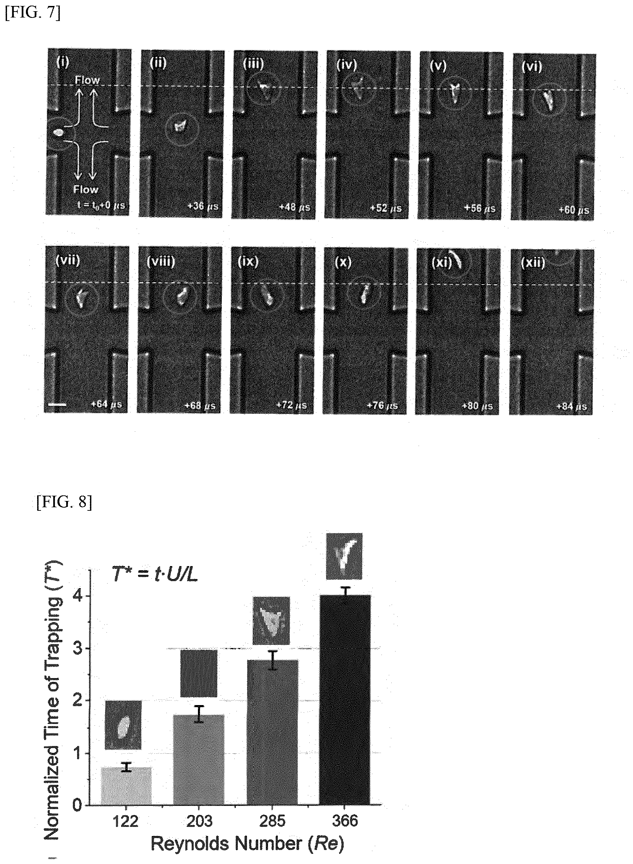

FIG. 7 shows high-speed microscopy images showing a cell (MDA-MB-231) trapping behavior in the + junction channel.

Referring to FIG. 7, a specific trapping phenomenon of cells is observed for a certain period of time as the cells leave a cell deformation region by the first vortex. That is, slightly above the stagnation point, the cells repeatedly move up and down for approximately 30 $\mu$s and then go out again toward the outlet. This means that immediately after leaving a vortex region, cells are deformed for a certain period of time and then stay in a region near the junction (a region near the stagnation point) by the inertia caused by the vortex breakdown, and thus the intracellular material delivery efficiency may be greatly improved.

FIG. 8 shows a graph of normalizing cell trapping and cell deformation time by vortex breakdown of FIG. 7 as a function of the Reynolds number.

Referring to FIG. 8, with the increase in the Reynolds number, the cell trapping and deformation times increase, and cell deformation for a certain period of time after the formation of the vortex can greatly improve the intracellular material delivery efficiency.

More specifically, the + junction structure microfluidic system according to the present disclosure will be described.

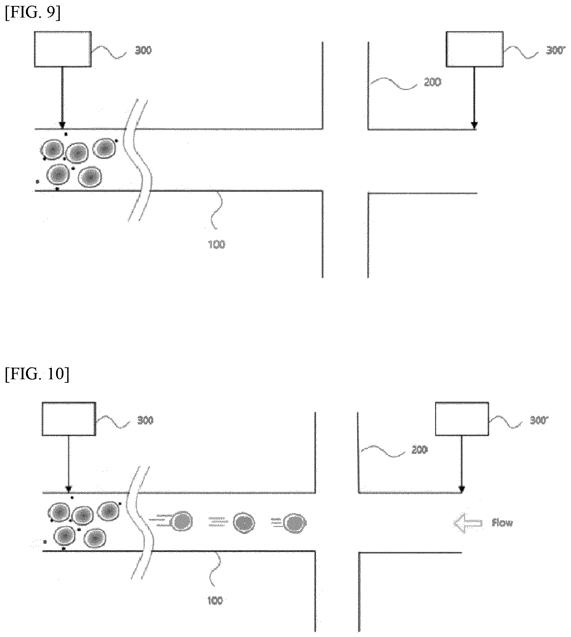

FIG. 9 is a diagram for illustrating an intracellular material delivery platform according to an embodiment of the present disclosure.

Referring to FIG. 9, the intracellular material delivery platform according to an embodiment of the present disclosure includes: a first channel 100 through which a fluid including cells and delivery material flows; a second channel 200 that vertically intersects with the first channel 100; and a first fluid control **unit** 300 provided on one side of the first channel 100 to control a fluid velocity in the first channel 100 in a first direction.

According to an embodiment of the present disclosure, the fluid in the first channel 100 flows in opposite directions to the point where the first channel 100 vertically intersects with the second channel 200, and the first fluid control **unit** 300 applies, to cells in the first channel 100, kinetic energy for causing cell membrane deformation at a level at which nanopores are formed in the cells by the vortex formed at the point where the first channel 100 and the second channel 200 vertically intersect each other.

In addition to that, a second fluid control **unit** 300' for controlling the fluid velocity in the first channel 100 in a second direction may be further included on the other side of the first channel 100.

In particular, in the first channel 100, the vortex may be formed at the point where the first channel 100 and the second channel 200 vertically intersect each other by the first and second fluid control units 300 and 300' performing controls in opposite directions, and due to the inertial force and inertial flow that are generated in this way, physical deformation may occur in the cell and the cell membrane may be deformed accordingly.

Meanwhile, the intracellular material delivery platform according to the present disclosure may deliver nucleic acids, proteins, transcription factors, vectors, plasmids, genetic-scissors materials, nanoparticles, and the like. However, the present disclosure is not limited thereto.

Furthermore, the intracellular material delivery platform is not limited in application to regenerative medicine, cancer immunotherapy, genomic editing, or other fields.

FIGS. 10 to 12 are diagrams for illustrating a method for intracellular delivery of materials using the intracellular material delivery platform according to an embodiment of the present disclosure.

Referring to FIG. 10, the fluid containing cells and delivery material flows in the first channel 100 by the first fluid control **unit** 300. At this time, the second fluid control **unit** 300' formed on the other side of the first channel 100 also operates such that the delivery material flows in a direction opposite to the fluid controlled by the first fluid control **unit** 300.

In an embodiment of the present disclosure, the delivery material includes all materials that may be delivered into cells, and specifically, genetic-scissors materials, plasmids, nucleic acids, proteins, nanoparticles, and the like may all the delivery materials.

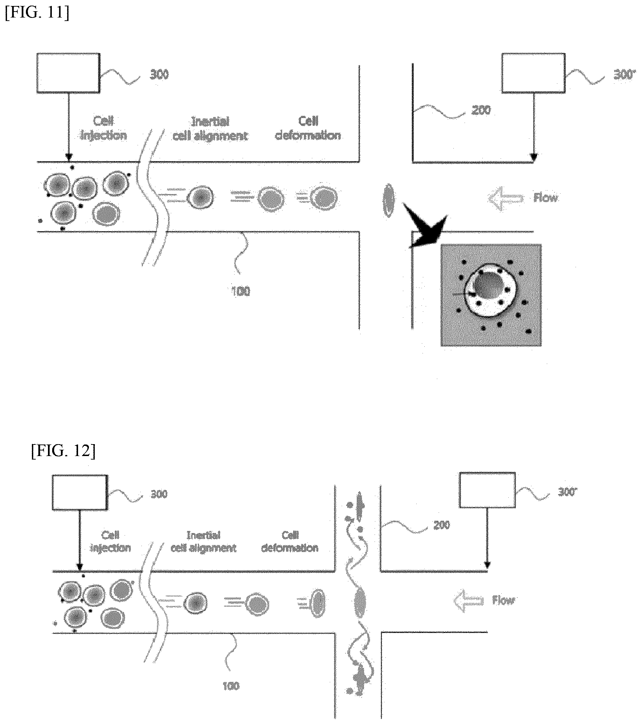

Referring to FIG. 11, the fluid accelerated by the first fluid control **unit** 300 forms a vortex along the interface with the opposing fluid near the junction, and the cells trapped by the vortex are deformed. Then, a nanopore is formed in the cell by the cell deformation. The delivery material is delivered into the cell through the nanopore. Accordingly, it is desirable that the first and second fluid control units 300 and 300' apply kinetic energy having a Reynolds number of a level at which the vortex of the fluid is formed near the junction.

According to another embodiment of the present disclosure, vortex breakdown formed after passing through the vortex formed in the fluid makes another cell deformation.

Referring to FIG. 12, cells passing through the vortex experience the vortex breakdown, and cell deformation occurs again due to inertia. Through the nanopore formed in the cell membrane by another cell deformation occurring at this time, the delivery material is again delivered into the cell, which greatly improves intracellular material delivery efficiency.

Embodiment 2: T or Y Junction Structure Microfluidic System

Another embodiment of the present disclosure provides an intracellular material delivery system using a T or Y-junction channel having a cavity. In the present disclosure, a cavity refers to an empty space formed in a channel at a stagnation point, which is a structure in which when the cells of the solution and the channel wall collide at the junction, a collision area between the cells and a channel wall is eliminated or reduced.

In this case, similar to the + junction channel described above, a localized vortex is formed near the T-junction, and sequentially, cell deformation, formation of the nanopore in the cell membrane, intracellular delivery of external materials, and closure of the nanopore in the cell membrane are performed.

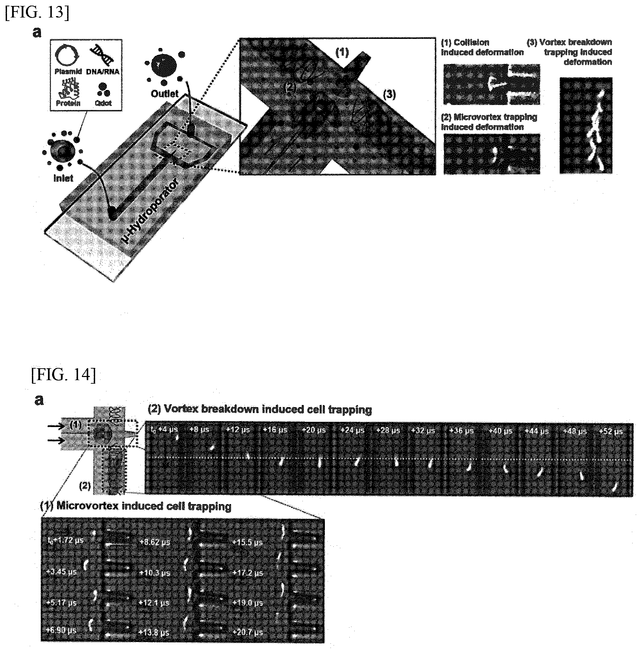

FIG. 13 is a schematic diagram of a T-junction channel according to an embodiment of the present disclosure.

Referring to FIG. 13, it can be seen that a channel-shaped cavity 1 is formed near a junction of a T-channel. In an embodiment of the present disclosure, cells mixed with external materials delivered into the cells are injected into the microfluidic channel of FIG. 13 by using a syringe pump in the related art (PHD 2000, Harvard Apparatus, USA), as a fluid control **unit**, at an appropriate Reynolds number.

Upon injection, the cells are concentrated in the channel center by inertia, and the cells collided with the channel wall. Each cell penetrates a portion of the above-mentioned cavity ((1) of FIG. 13) and is deformed (refer to the photographs on the right of FIG. 13). Then, after the first cell deformation by the collision, the cell is trapped in a localized vortex near the stagnation point (point (2) in FIG. 13) and is hydrodynamically deformed to form nanopores in the cell membrane, as described in FIG. 3.

Then, the cell that has passed through the vortex region are trapped and then deformed by vortex breakdown again ((3) of Fig. 13). An advantage of such additional cell trapping and deformation is the improvement of intracellular material delivery efficiency as described above in FIG. 7.

In an embodiment of the present disclosure, the cavity is used in the T-junction channel structure, the advantage of which is to reduce cell damage due to collision with a rigid solid channel wall by allowing cells to collide with the channel wall of a fluid form, instead of the channel wall of a rigid solid form. Another advantage is to virtually prevent cell clogging by creating a stagnation point upstream in the fluid flow direction to support complex fluid behavior patterns. Accordingly, the form, size, shape, and the like of the cavity structure may vary depending on the cell. For example, the cavity may include not only an elongated slit structure as shown in FIG. 13 but also a circular, oval, elongated slit, square, rectangular, trapezoidal, polygonal, a combination thereof, and a modified form thereof, and at least as long as the introduced cells do not collide with the channel wall as they are, they all belong to the cavity of the present disclosure.

In addition, the cavity diameter is determined depending on the cell diameter, and in particular, the cavity diameter is preferably on the order of 10% to 5 times the cell size. The cavity diameter according to the present disclosure is within the scope of the present disclosure, at least as long as the cavity diameter can reduce the collision force caused by the collision between the cells, which are introduced to the junction through the solution, and the channel wall.

FIG. 14 shows high-speed microscopy images illustrating a cell deformation and vortex deformation mechanism of a T-junction channel of a cavity structure. Here, each arrow indicates a fluid flow direction.

Referring to FIG. 14, trapping by the localized vortex near the stagnation point ((1) in FIG. 14) and trapping of the cell passing through the stagnation point by vortex breakdown due to fluid instability near the stagnation point ((2) in FIG. 14) can be confirmed, which shows that the vortex, the trapping by the vortex breakdown, and the deformation occur continuously, similar to the channel of the + channel described above. In particular, in (2) of FIG. 14, it can be seen that the cell maintains a static state for about 20 $\mu$s and then exits downstream.

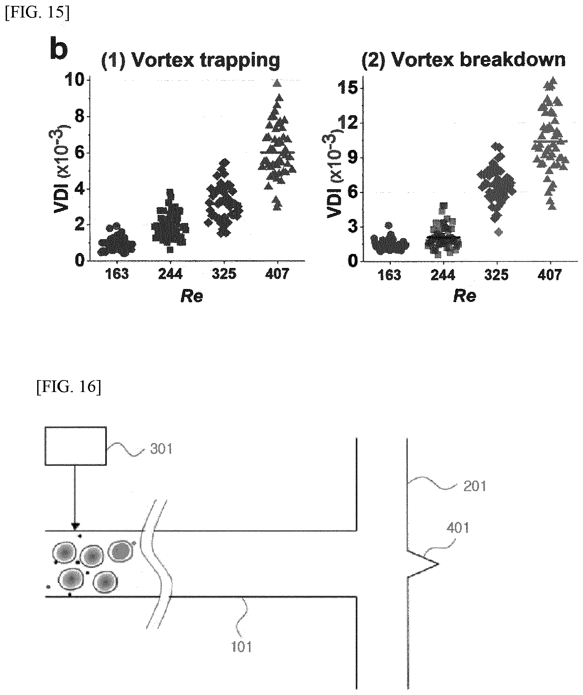

FIG. 15 shows a vortex deformability index (VDI) analysis result in a fluid having different Reynolds numbers. Here, VDI is defined as the following formula, which can be interpreted as the degree of cell deformability index and the duration of cell membrane permeation, which in turn indicates the intracellular material delivery efficiency.

$$VDI = t(1-c)U/D$$

where t is the cell trapping time in the vortex, c is the

circularity ($c=4\pi A/P^2$, where A and P are the area and radius in the state with maximum deformation, respectively), U is the average velocity of the fluid, and D is the cell diameter.

Referring to FIG. 15, with the increase in the Reynolds number, the VDI increases during (1) the vortex and (2) the vortex breakdown, indicating that a high Reynolds number has a higher intracellular material delivery efficiency.

Hereinafter, the T-junction structure microfluidic system according to the present disclosure will be described in more detail.

FIG. 16 is a diagram for illustrating an intracellular material delivery platform according to an embodiment of the present disclosure.

The intracellular material delivery platform according to the present disclosure includes: a third channel 101 forming a pathway through which a fluid including cells and delivery material moves; a fourth channel 201 vertically extending to both sides of the third channel 101 at an end of the third channel 101; and a fluid control **unit** 301 provided at the third channel 101 to control a fluid velocity in the third channel 101.

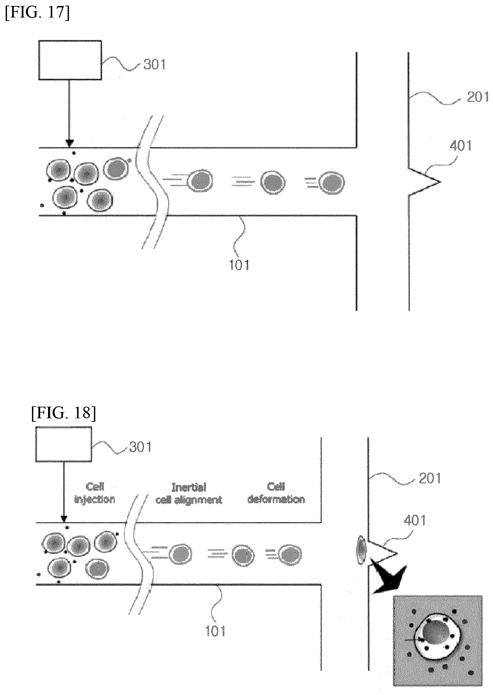

FIGS. 17 to 19 are diagrams for illustrating a method for intracellular delivery of materials using the intracellular material delivery platform according to an embodiment of the present disclosure.

Referring to FIG. 17, the fluid containing cells and delivery material flows in the third channel 101 by the fluid control **unit** 301. In an embodiment of the present disclosure, the delivery material includes all materials that may be delivered into cells, and genetic-scissors materials, plasmids, nucleic acids, proteins, nanoparticles, and the like are all the delivery materials.

Referring to FIG. 18, the cells in the third channel 101 accelerated by the fluid control **unit** 301 are trapped by the vortex formed near the junction and then deformed. This is the same as that described with reference to FIGS. 13 and 14. Then, the cells collide with a partition wall of the fourth channel 201 connected to the end of the third channel 101.

At this time, the fourth channel 201 is provided with a slit-shaped cavity 401 formed in the same direction as the fluid flow direction in the third channel 101, and the cavity produces effects of 1) preventing cell damage by physical collision, 2) preventing clogging, and 3) forming the stagnation point upstream.

In the present disclosure, as described above, a plurality of unit microfluidic systems may be connected in series or parallel or a combination thereof to construct an entire system. In this case, the vortex may occur in the recirculated stream in a circulation mode, where the vortex may be a closed or open stream.

Experimental examples

Experimental Example 1

In the experimental example, delivery characteristics of the microfluidic system based on Embodiment 1 were analyzed.

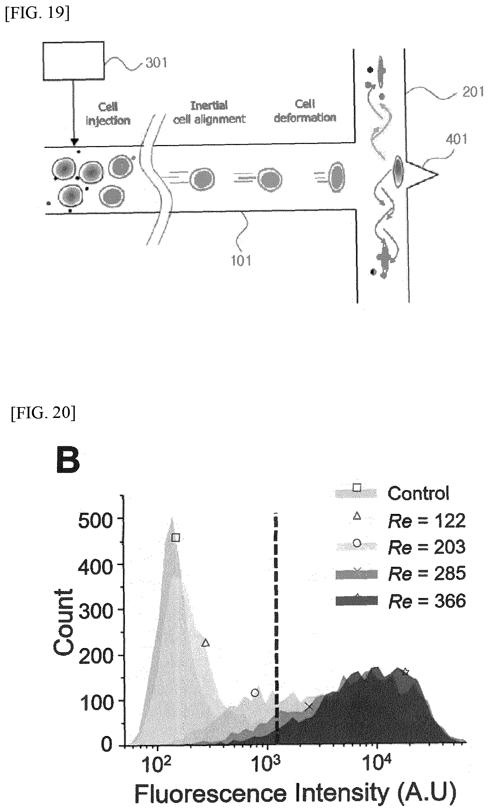

FIG. 19 shows a result of comparing the intracellular material delivery efficiencies after 18 hours by the endocytosis (control group) and the intracellular mass transfer method according to the present disclosure (spiral hydroporation). Here, 3-5 kDa fluorescein isothiocyanate (FITC)-conjugated dextran was delivered into MDA-MB-231 cells, and the result was shown as a fluorescence image (scale bar: 40 $\mu$m). In particular, it should be noted here that the delivery of dextran into MDA-MB-231 cells is known to be very difficult. In the experiments described below, unless otherwise noted, cells are MDA-MB-231.

Referring to FIG. 19, the intracellular material delivery effect according to the present disclosure can be confirmed through multiple FITC signals on the right.

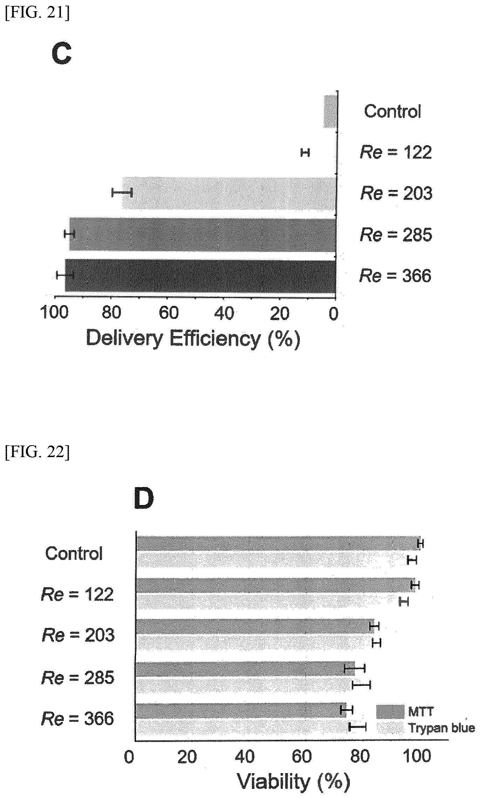

FIGS. 20 and 21 show analysis results of the amount of materials delivered into cells. In the analysis, the delivery efficiency was defined as a fraction of fluorescence signals equal to or greater than 5%, which corresponds to the red dotted line.

Referring to FIGS. 20 and 21, it can be seen that at Reynolds number 366, a delivery efficiency of approximately 96.5% was achieved, and the fluorescence intensity increases with the increase in Re and the histogram profile shifted to the right. This means that the amount of materials delivered into cells may be adjusted by adjusting the Reynolds number.

FIG. 22 shows a result of measuring cell viability by varying the Reynolds number under the same conditions as in FIG. 19.

Referring to FIG. 22, with regard to cell viability, a decrease in viability was observed at a higher Reynolds number, presumably because the higher the Reynolds number, the greater the cell perturbation.

Furthermore, in order to further investigate cell viability, a standard MTT assay was performed via metabolic function and the result is shown in FIG. 22. The overall trends of the trypan blue exclusion method and the MTT assay were in good agreement with each other, whereas the MTT assay showed slightly lower viability values at high Reynolds numbers.

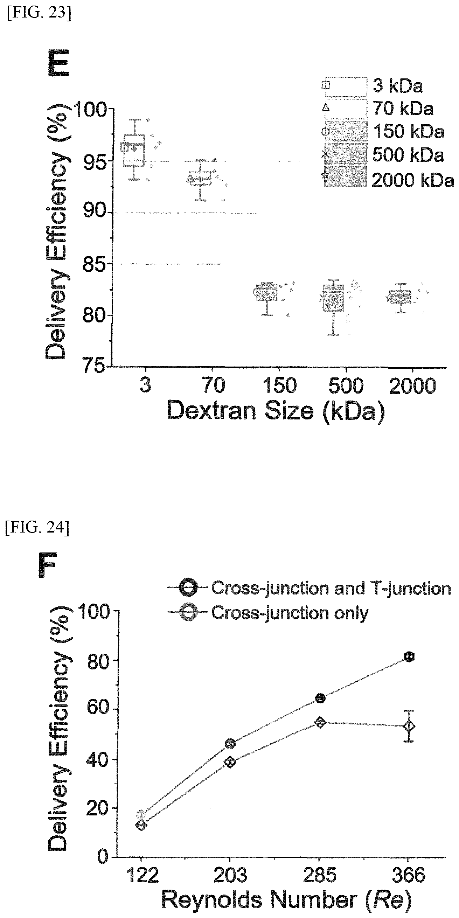

FIG. 23 shows an analysis result of delivery efficiency for various dextran sizes. In the analysis, dextrans of molecular weights ranging from 3 to 2000 kDa, corresponding to a hydraulic diameter of 2 to 55 nm, were used, where FITC-dextrans (3 to 5, 70, 150, 500, and 2000 kDa) of 5 different sizes and the same concentration were delivered into MDA-MB-231 cells in the same manner as in Embodiment 1 under the same flow conditions (Re = 366) and the efficiency was calculated.

Referring to FIG. 23, it was shown that relatively small dextran (<70 kDa) had higher delivery efficiency than relatively large dextran. This is because, for small dextran, convective and diffuse transport of dextran across the cell membrane occurs, whereas for large dextran, convective transport is the dominant transport.

FIG. 24 shows a result of measuring dextran delivery efficiency in the complex microfluidic system of FIG. 5 (a combination of the + junction channel and the T-junction channel) .

Referring to FIG. 24, it can be seen that the complex microfluidic system of FIG. 5, in which continuous cell deformation was performed, exhibited relatively higher delivery efficiency than the stand-alone one.

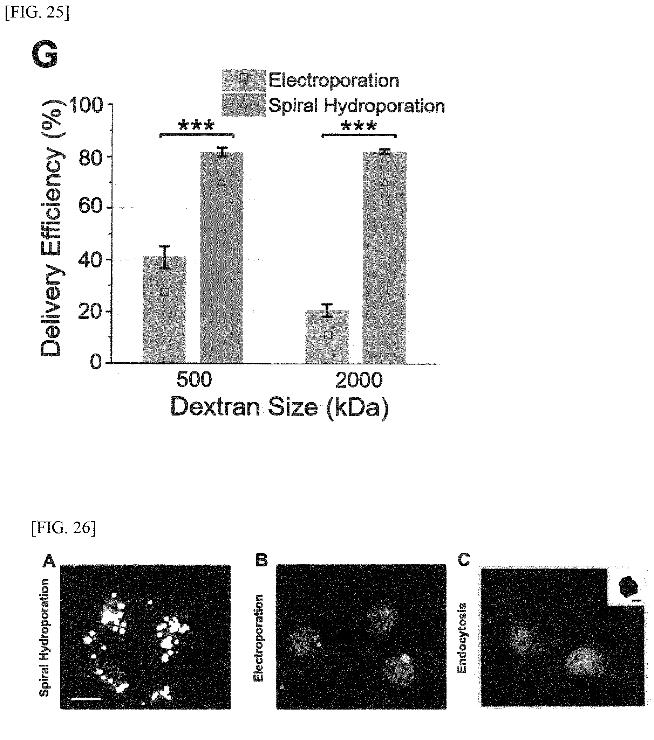

FIG. 25 shows a comparison result of the delivery efficiency of electroporation in the related art (a neon transfection system (Thermo Fisher Scientific, Waltham, MA, USA)) and the method for delivery of materials based on inertia (hydroporation) according to the present disclosure. In the experimental example, 500 and 2000 kDa FITC-dextrans were used as external materials.

Referring to FIG. 25, for the 2000 kDa FITC-dextran, the efficiency of the present disclosure was approximately 4 times higher than that of electroporation. In particular, the method according to the present disclosure greatly improves the delivery efficiency of macromolecules that are difficult to be delivered into cells by electroporation, which suggests the possibility of intracellular delivery of molecular weight materials that is not possible to be achieved by electroporation techniques in the related art.

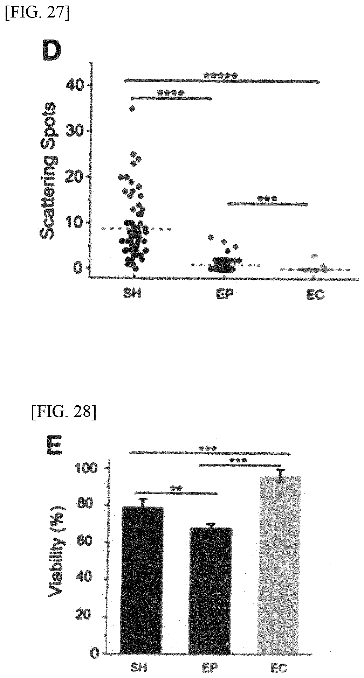

FIGS. 26 and 27 are photographs for analyzing the intracellular delivery effect of gold nanoparticles (GNPs), and a result of counting scattering spots, respectively. Here, (A) shows a result of hydroporation (SH) according to the present disclosure, (B) shows a result of electroporation (EP), and (C) shows a result of endocytosis (EC).

Referring to FIGS. 26 and 27, it can be seen that very large GNPs (200 nm in diameter) were successfully delivered into MDAMB-231 cells through the fluidic system according to the present disclosure (A). On the other hand, it can be seen that only a few scattering points were detected although electroporation was able to deliver GNPs (B). Furthermore, the endocytosis mechanism exhibited lower particle delivery efficiency than that of electroporation (C).

FIG. 28 shows a result of measuring cell viability depending on GNP delivery.

Referring to FIG. 28, it can be seen that the method and system for intracellular delivery of materials according to the present disclosure exhibited higher viability than that of electroporation.

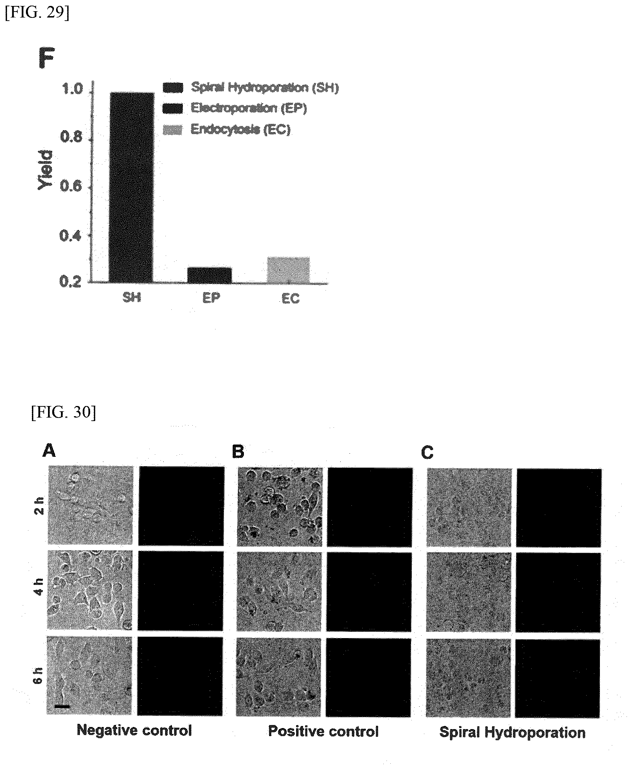

FIG. 29 shows a result of calculating a yield normalized by multiplying the number of scattering spots by the cell viability (FIG. 5F).

Referring to FIG. 29, it can be seen that the material delivery efficiency according to the present disclosure was three times higher than that of electroporation or endocytosis.

FIG. 30 shows an analysis result of intracellular delivery efficiency of mesoporous silica nanoparticles (MSN) used as a drug, protein, and nucleic acid nanocarrier instead of gold nanoparticles. In the experiment, doxorubicin (DOX), an anticancer drug, was loaded into MSNs and the MSNs were delivered into MDA-MB-231 cells.

Referring to FIG. 30, it can be seen that the method according to the present disclosure (spiral control) exhibited a number of bright fluorescence points compared to endocytosis. This suggests that a carrier carrying a functional material can be effectively delivered into the cell by the method according to the present disclosure.

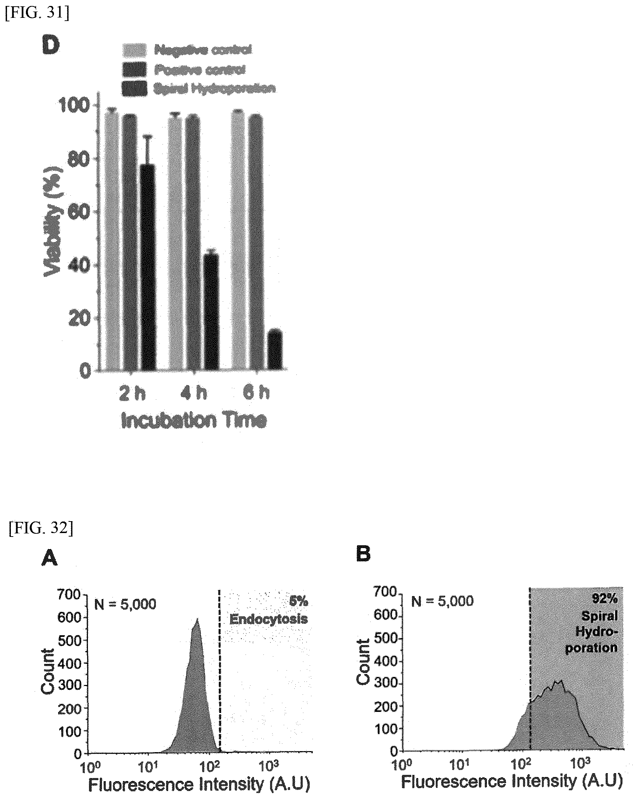

FIG. 31 shows an analysis result of a time-dependent cell viability for the analyte of FIG. 30.

Referring to FIG. 31, DOX cytotoxicity to MDA-MB-231 cells was measured by the trypan blue exclusion method, and it can be seen that approximately 85% of cancer cells were killed after 6 hours. The result indicates that the microfluidic system according to the present disclosure may investigate DOX-induced cell death without using a surface ligand-based DOX delivery method in the related art.

FIG. 32 is a histogram of fluorescence intensity obtained by measuring a delivery effect to K562 cells using mRNA (996-nucleotide mRNA fragment, green fluorescent protein) as an external material. In FIG. 32, (A) shows a result of endocytosis and (B) shows a result of the microfluidic system according to the present disclosure, where EGFP protein expression was assessed based on mRNA delivery (2 pg/mL) using a flow cytometer.

Referring to FIG. 32, it can be seen that the method according to the present disclosure exhibited a very high protein expression level. This means that the method and system for intracellular delivery of materials according to the present disclosure may be used for chimeric antigen receptor-expressing T-cells (CAR-T) without the use of vectors or vaccines.

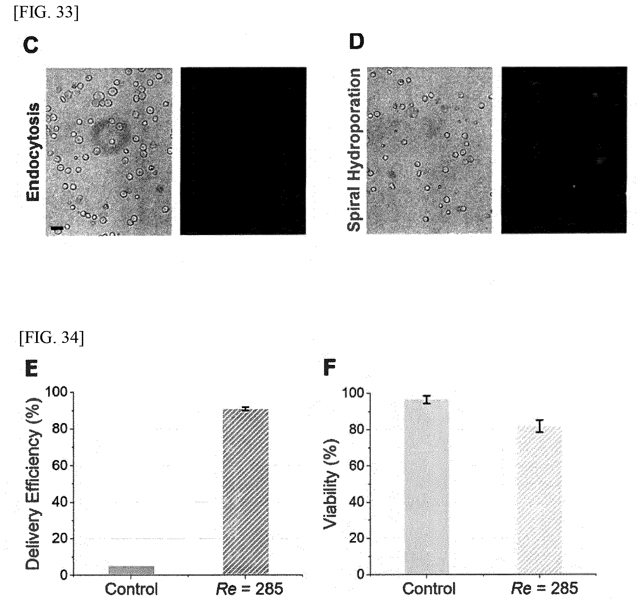

FIG. 33 shows fluorescence images after delivery of mRNA into K562 by (C) endocytosis and (D) the microfluidic system according to the present disclosure.

Referring to FIG. 33, in the method according to the present disclosure, strong EGFP signals were detected compared to endocytosis, which results in higher mRNA delivery efficiency.

FIG. 34 shows a result of measuring delivery efficiency (E) and viability (F) when mRNA is delivered according to the present disclosure as shown in FIGS. 32 and 33.

Referring to FIG. 34, the delivery efficiency of up to approximately 92% was achieved without sacrificing cell viability, which represents the high potential of the platform for use of the platform for immunotherapy research although further investigations are needed to test human immune cells in the future.

Experimental Example 2

In the experimental example, delivery characteristics of the microfluidic system (μ-Hydroporator) based on Embodiment 2 were analyzed.

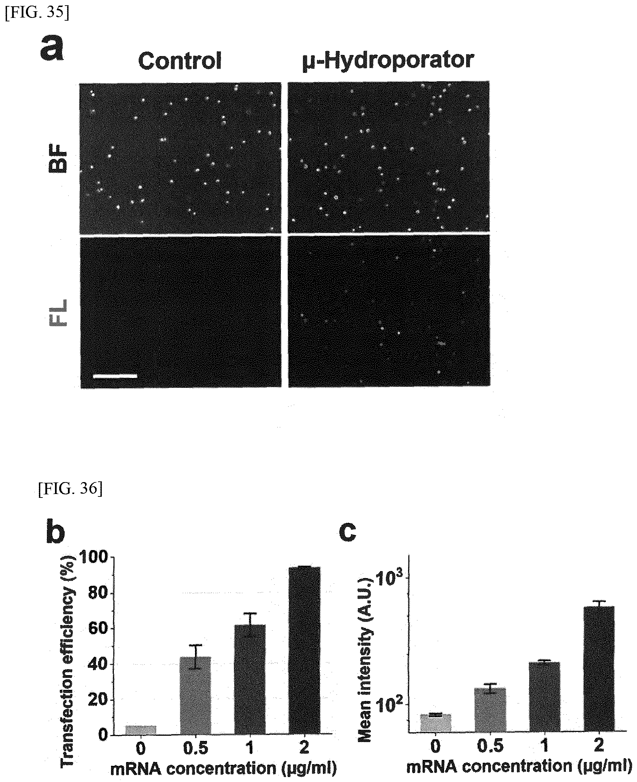

FIG. 35 shows fluorescence images after mRNA (996-nucleotide mRNA fragment, green fluorescent protein) is delivered into K562 cells by endocytosis and the method of Embodiment 2.

Referring to FIG. 35, it can be seen that, unlike endocytosis (control), green fluorescence was detected when mRNA was delivered into cells by the method of Embodiment 2. This proves that the T-junction channel system according to the present disclosure (Embodiment 2) enables intracellular delivery of mRNA with high efficiency as in Embodiment 1 described above.

FIG. 36 shows an analysis result of (b) mRNA transfection efficiency and (c) mean fluorescence intensity depending on mRNA concentration.

Referring to FIG. 36, different concentrations of mRNAs were accessed based on flow cytometry analysis, and in 2 pg/ml, transfection efficiency was achieved up to approximately 93%. This is an efficiency that is not possible in compression-based microfluidic systems such as bottleneck structures in the related art.

Unlike mRNA, which binds to the cell substrate first, DNA has to first pass through the cell membrane and enter a nuclear membrane through a nuclear pore. Furthermore, in terms of material delivery, naked plasmid DNA has a drawback in that it is easily degraded by nucleases and has a high viscosity. In addition, high-density cytoplasm does not provide favorable conditions for long, twisted DNA to reach the nucleus purely by diffusion. In order to overcome the above-mentioned drawback, the present inventors provide a fluid-based microfluidic system according to the present disclosure as a method for delivering plasmid DNA to a nucleus. To this end, in the experimental example, an experiment was performed to encode copepod GFP and deliver 7.9 kbp plasmid DNA to HEK293t cells.

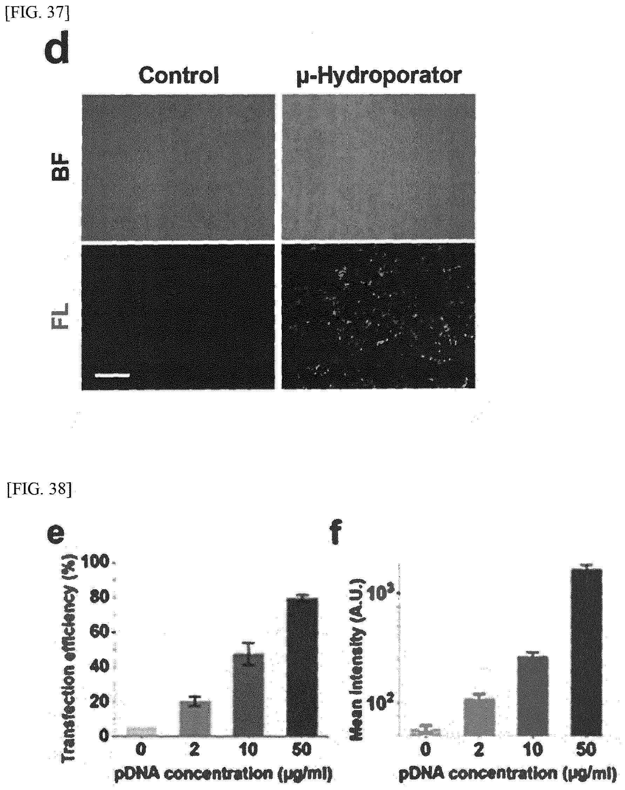

FIG. 37 shows fluorescence images after delivery of plasmid DNA into HEK293t by the endocytosis (control) and the method of Embodiment 2.

Referring to FIG. 37, it can be seen that the method according to the present disclosure exhibited a very strong fluorescence image.

FIG. 38 shows a graph of analyzing plasmid DNA (pDNA) transfection efficiency (E) of HEK293t cells and mean intensity (F) of HEK293t cells depending on plasmid DNA concentration.

Referring to FIG. 38, it can be seen that with an increase in the pDNA concentration, the transfection efficiency increases, and the mean fluorescence intensity also increases.

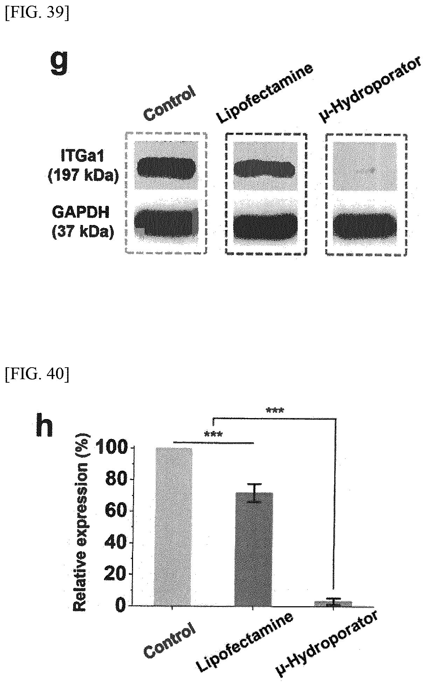

FIGS. 39 and 40 are a result of western blot analysis of the ITGa1 gene knockdown effect of HeLa cells (α1 subunit of an integrin transmembrane receptor) into which siRNA is delivered, and a result of comparing relative expression levels, respectively. Here, the knockdown effect of the ITGa1 gene was compared using cationic Lipofectamine 3000 in the related art, which is known as the siRNA delivery method, as a comparative example.

Referring to FIGS. 39 and 40, when the present disclosure was used, ITGA1 expression (197 kDa) was almost eliminated; whereas, in Lipofectamine 3000, the comparative example, only partial knockdown was observed. According to the result, the present disclosure has at least three times greater knockdown efficiency (97% vs. 30%), suggesting that the present disclosure has a high potential for use in gene editing.

In an experiment below, non-functional materials or extremely small molecules (e.g., calcein, propidium iodide, and 3 kDa dextran) were delivered to cell lines. In the experiment below, mRNA was chosen as a delivery target, since protein expression after mRNA delivery occurs in the cytoplasm and is guided to fat, is well controllable, and is easily comparable by dose-dependent transfection.

In the experiment, EGFP mRNA was delivered into Harton's jelly human umbilical cord mesenchymal stem cells (MSCs), human adipose derived stem cells (ADSCs), and mouse bone marrow derived dendritic cells (BMDC) by using the method of Embodiment 2 (electroporator), Lipofectamine 3000, and the electroporation neon transfection system (electroporator; Thermo Fisher Scientific).

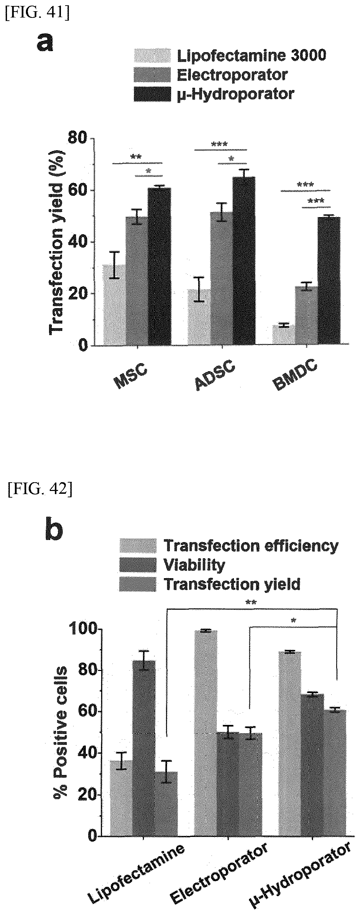

FIG. 41 shows an analysis result of transfection yields by Lipofectamin 3000, electroporation, and the present disclosure.

Referring to FIG. 41, it can be seen that the present disclosure exhibited a very high transfection yield compared to Lipofectamine 3000 and electroporation. In the analysis result, the transfection yield was defined as the product of transfection efficiency and cell viability, which can be understood as the ratio of viable cells to cells transfected by material delivery.

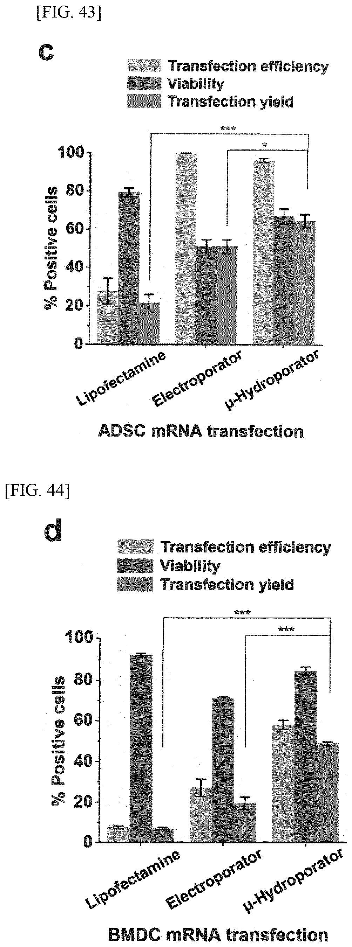

FIGS. 42 to 44 are analysis results of transfection efficiencies, cell viability, and transfection yields for human MSC, human ADSC, and mouse BMDC.

Referring to FIGS. 42 to 44, it can be seen that lipofection exhibited improved cell viability in all cell types compared to the present disclosure (μ-Hydroporator) and electroporation, but exhibited substantially low transfection efficiency in all cell types.

In addition, for MSC and ADSC, the transfection efficiency was slightly higher in the electroporator than in the present disclosure (μ-Hydroporation). However, for all cell types, the present disclosure (μ-Hydroporator) exhibited higher cell viability without the use of special stabilized buffer required for the electroporator. Furthermore, the cell viability of the present disclosure may further increase cell viability simply by adding trehalose or polymer to the cell media.

For BMDCs, the present disclosure exhibited higher transfection efficiency and cell viability than electroporation, indicating that the present disclosure has a high potential to be used for cancer immunotherapy.

The present disclosure has several advantages over electroporation in the related art with respect to immune cell therapy. First, electroporation is known to have a side effect of altering important properties of primary T cells (e.g., non-specific cytokine burst and blunted IFN-$\gamma$ response), lowering the therapeutic performance. However, the low scalability of electroporation (treating $10^4$ to $10^5$ cells per run) is a drawback regarding its potential clinical usage in cancer immunotherapy, which generally requires the treatment of $10^8$ cells.

However, in the present disclosure, $1 \times 10^6$ cells/min may be processed while the same level of delivery efficiency is maintained and this throughput is based on a single microchannel, and thus the present disclosure may achieve the cell throughput required for cancer immunotherapy through multiplexing and parallelization of microchannels.

In an experiment below, quantum dots (Dibenzo cyclooctyne (DOBI)) and silica nanospheres, which are widely used as target molecules, were determined as intracellular delivery materials, and delivery properties to cells (MDA-MB-231) were analyzed.

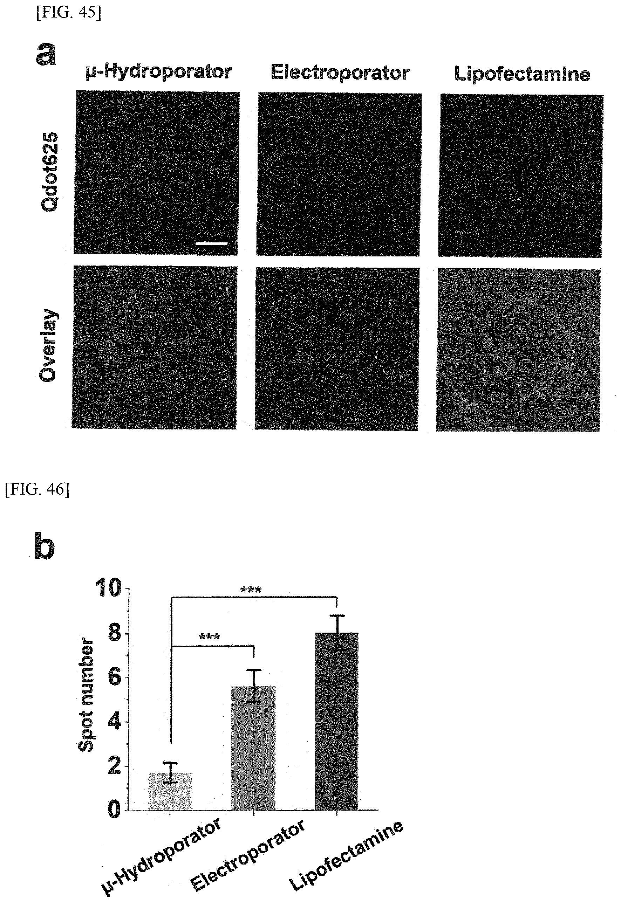

FIG. 45 shows confocal microscopy images showing delivery of quantum dots (qdot625) into MDA-MB-231 cells by the present disclosure ($\mu$-Hydroporator), electroporator, and Lipofectamine 3000.

Referring to FIG. 45, all the methods showed excellent delivery efficiency, but the result in which quantum dots were well dispersed in the cytoplasm was shown when intracellular delivery was performed by the method according to the present disclosure. In cells treated with electroporation and Lipofectamine 3000, multiple red spots were observed, indicating the possibility of aggregation or endosome entrapment of the quantum dots. Since such quantum dot aggregation eventually causes a decrease in the efficiency of intracellular delivery of materials, the result means that the method for intracellular delivery of materials according to the present disclosure is also useful for intracellular delivery of micromaterials.

FIG. 46 shows an analysis result of spot number counts of quantum dots (Qdot 625) per cell.

Referring to FIG. 46, the analysis result represents the degree of aggregation of quantum dots, and it can be seen that in the present disclosure, the number of quantum dots per cell was the lowest. That is, electroporation or lipofection exhibited three- and four-fold levels of quantum dot count compared to the present disclosure. This is also consistent with the analysis result of FIG. 45, indicating that when intracellular delivery of particles such as quantum dots is performed according to the present disclosure, quantum dots are well dispersed in the cytoplasm by rapid solution exchange through the cell membrane after cell deformation.

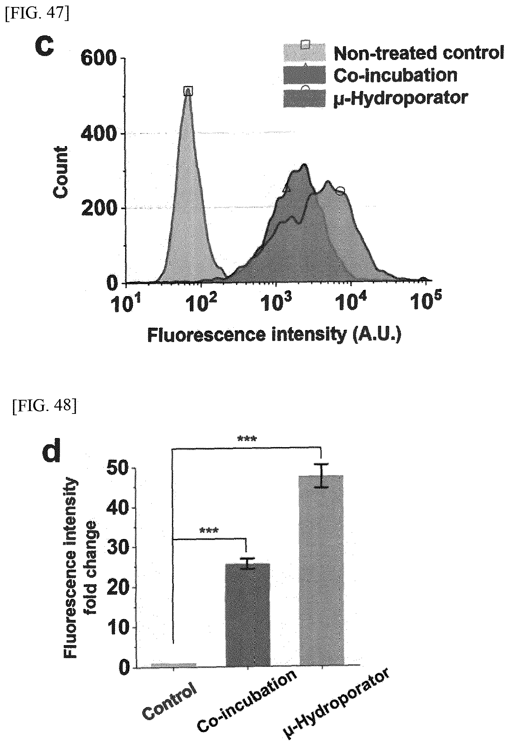

FIG. 47 is a fluorescence intensity histogram of Embodiment ($\mu$-Hydroporator, $N_{cell}$ = 5,000 per sample) in which nanospheres (green fluorescent silica nanospheres, Micromod, Germany) are delivered into K562 cells, by a negative control (nontreated K562 cells), a positive control (K562 cells co-incubated with nanospheres, the same time and concentration as those in Embodiment), and the present disclosure ($\mu$-Hydroporator).

Further, FIG. 48 shows a result of measuring relative mean fluorescence intensity.

Referring to FIGS. 47 and 48, on average, higher fluorescence intensity was observed in cells treated according to the present disclosure, but high fluorescence signals were detected in the co-incubation group in flow cytometry analysis. Considering the short incubation time and the excessively large nanosphere size for endocytosis, the fluorescence in the positive control presumably seems to be obtained from nanospheres adhering to the cell surfaces.

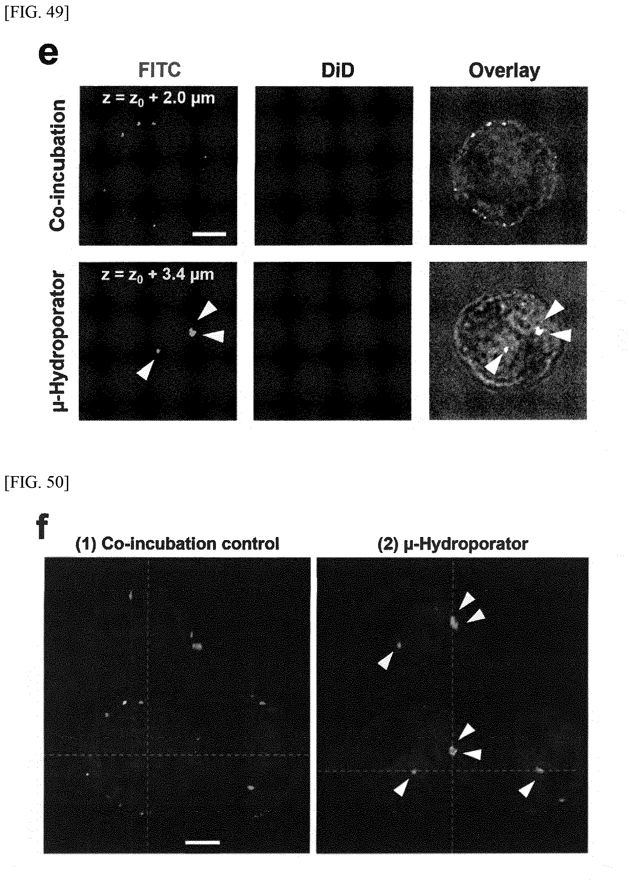

FIGS. 49 and 50 show confocal images of two of the groups of FIGS. 47 and 48. Here, the cell membrane was visualized by using DiD lipophilic carbocyanine dye.

As shown in FIGS. 49 and 50, the green fluorescence signals from the positive control (co-incubation) were present only in the cell membrane, whereas bright green fluorescence from the cytoplasm was only observed in cells treated according to the present disclosure. This indicates that only the system for intracellular delivery of materials according to the present disclosure is possible as a technology capable of delivering nanospheres into cells, considering the positive control in which only the action of nanospheres adhering to the cell surfaces by electrostatics is confirmed.

## INDUSTRIAL APPLICABILITY

The microfluidic system for delivering external materials into cells by cell mechanoporation using inertia according to the present disclosure has industrial applicability in the bio and medicine fields requiring delivery of materials into cells.

## Claims

1. A microfluidic system delivering external materials into a cell by cell mechanoporation using inertia, the microfluidic system comprising:

   a fluidic channel structure through which a solution containing a cell and external materials flows continuously,
   wherein the fluidic channel structure includes a junction between one or more channels,
   a localized vortex is generated near an interface of the junction,
   the cell is deformed by the vortex, and
   transient discontinuities are generated in a cell membrane by the vortex and the external materials are introduced into the cell by solution exchange between the cell and fluid around the cell.

2. The microfluidic system of claim 1, wherein the fluidic channel structure including the junction between one or more channels includes a junction including a T, Y, cross shape, or a combination thereof.

3. The microfluidic system of claim 2, wherein the fluidic channel structure includes a cavity near a fluid stagnation point when the fluidic channel structure is a channel of the T or Y shape.

4. The microfluidic system of claim 3, wherein the cavity has a shape of a circle, an ellipse, an elongate slit, a square, a rectangle, a trapezoid, a polygon, and a combination thereof, and a modification thereof.

5. The microfluidic system of claim 3, wherein a diameter of the cavity is determined according to a diameter of the cell.

6. The microfluidic system of claim 3, wherein the cavity has a structure for eliminating or reducing a collision area between the cell and a channel wall when the cell of the solution collides with the channel wall at the junction.

7. The microfluidic system of any one of claims 1 to 6, further comprising a fluid control unit for allowing a solution to flow in the fluidic channel structure, wherein the fluid control unit allows the solution to flow in the fluidic channel at a velocity that is at a level capable of generating a localized vortex near the interface of the junction.

8. The microfluidic system of claim 7, wherein the fluid control unit is a syringe pump or pneumatic system.

9. The microfluidic system of any one of claims 1 to 8, wherein a Reynolds number (Re) of the solution is 1 to 1000.

10. The microfluidic system of claim 9, wherein the vortex is determined by the Reynolds number.

11. The microfluidic system of any one of claims 1 to 8, wherein the vortex is in a form of a closed or open recirculating flow.

12. The microfluidic system of any one of claims 1 to 8, wherein the fluidic channel has a plurality of the junctions at least in a channel between an inlet and an outlet of the solution.

13. The microfluidic system of claim 10, wherein the microfluidic system is formed by combining the microfluidic system according to any one of claims 1 to 12 in series, parallel, or a combination thereof.

14. A method of delivering external materials into a cell by cell mechanoporation using inertia, the method comprising:

allowing a solution containing the cell and external materials to continuously flow a fluidic channel;
forming a vortex by a vortex generating means near the junction;
deforming the cell by the vortex; and
allowing the external materials to be introduced into the cell through a pore created in a cell membrane by the deforming of the cell.

15. The method of claim 14, wherein the vortex generating means is a junction structure of the fluidic channels.

16. The method of claim 15, wherein the fluidic channel includes a junction including a T, Y, cross shape, or a combination thereof.

EP 3 939 701 A1

[FIG. 1]

**A**  **Spiral Hydroporator**

[FIG. 2]

**B** (1) (2)

13

[FIG. 3]

[FIG. 4]

[FIG. 5]

[FIG. 6]

[FIG. 7]

[FIG. 8]

$$T^* = t \cdot U / L$$

[FIG. 9]

[FIG. 10]

[FIG. 11]

[FIG. 12]

[FIG. 13]

[FIG. 14]

[FIG. 15]

[FIG. 16]

[FIG. 17]

[FIG. 18]

Cell injection    Inertial cell alignment    Cell deformation

[FIG. 19]

[FIG. 20]

[FIG. 21]

[FIG. 22]

[FIG. 23]

[FIG. 24]

[FIG. 25]

[FIG. 26]

[FIG. 27]

[FIG. 28]

[FIG. 29]

[FIG. 30]

Negative control     Positive control     Spiral Hydroporation

[FIG. 31]

[FIG. 32]

[FIG. 33]

[FIG. 34]

[FIG. 35]

[FIG. 36]

[FIG. 37]

[FIG. 38]

[FIG. 39]

[FIG. 40]

[FIG. 41]

[FIG. 42]

[FIG. 43]

**ADSC mRNA transfection**

[FIG. 44]

**BMDC mRNA transfection**

[FIG. 45]

[FIG. 46]

[FIG. 47]

[FIG. 48]

[FIG. 49]

[FIG. 50]

## INTERNATIONAL SEARCH REPORT

| | |
|---|---|
| International application No. |
| **PCT/KR2020/003411** |

### A.  CLASSIFICATION OF SUBJECT MATTER

*B01L 3/00(2006.01)i*

According to International Patent Classification (IPC) or to both national classification and IPC

### B.  FIELDS SEARCHED

Minimum documentation searched (classification system followed by classification symbols)
B01L 3/00; B81C 99/00; C12N 1/00; C12N 13/00; C12N 15/89

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), GOOGLE & Keywords: intracellular delivery, vortex, T-junction, cavity

### C.  DOCUMENTS CONSIDERED TO BE RELEVANT

| Category* | Citation of document, with indication, where appropriate, of the relevant passages | Relevant to claim No. |
|---|---|---|
| Y | DENG, Y. et al. Intracellular Delivery of Nanomaterials via an Inertial Microfluidic Cell Hydroporator. NANOLETTERS. 2018, vol. 18, no. 4, pages 2705-2710 See pages 2705-2709; figure 1. | 1-8,14-16 |
| Y | JARRELL, J. A et al. Intracellular Delivery of mRNA to Human Primary T Cells With Microfluidic Vortex Shedding. SCIENTIFIC REPORTS. 2019 (Published online on 01 March 2019), vol. 9, no. 3214, pages 1-11 See abstract; pages 1-3; figure 1. | 1-8,14-16 |
| A | KR 10-1515394 B1 (INDUSTRY-ACADEMIC COOPERATION FOUNDATION, YONSEI UNIVERSITY) 04 May 2015 See the entire document. | 1-8,14-16 |
| A | KR 10-2014-0116374 A (MASSACHUSETTS INSTITUTE OF TECHNOLOGY) 02 October 2014 See the entire document. | 1-8,14-16 |
| A | JP 2016-526923 A (PRESIDENT AND FELLOWS OF HARVARD COLLEGE) 08 September 2016 See the entire document. | 1-8,14-16 |

☐  Further documents are listed in the continuation of Box C.       ☒  See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| 24 JUNE 2020 (24.06.2020) | **25 JUNE 2020 (25.06.2020)** |

| Name and mailing address of the ISA/KR <br> Korean Intellectual Property Office <br> Government Complex Daejeon Building 4, 189, Cheongsa-ro, Seo-gu, Daejeon, 35208, Republic of Korea <br> Facsimile No. +82-42-481-8578 | Authorized officer <br><br> Telephone No. |
|---|---|

Form PCT/ISA/210 (second sheet) (January 2015)

## INTERNATIONAL SEARCH REPORT

| International application No. |
| --- |
| **PCT/KR2020/003411** |

**Box No. II**     **Observations where certain claims were found unsearchable (Continuation of item 2 of first sheet)**

This international search report has not been established in respect of certain claims under Article 17(2)(a) for the following reasons:

1. ☐   Claims Nos.:
   because they relate to subject matter not required to be searched by this Authority, namely:

2. ☒   Claims Nos.: **10**
   because they relate to parts of the international application that do not comply with the prescribed requirements to such an extent that no meaningful international search can be carried out, specifically:

   Claim 10 refers to claim 9 which violates the manner of referring to a dependent claim (PCT Rule 6.4(a)), and thus is unclear.

3. ☒   Claims Nos.: **9, 11-13**
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

1. ☐   As all required additional search fees were timely paid by the applicant, this international search report covers all searchable claims.

2. ☐   As all searchable claims could be searched without effort justifying additional fees, this Authority did not invite payment of additional fees.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**     ☐   The additional search fees were accompanied by the applicant's protest and, where applicable, the payment of a protest fee.

                                                ☐   The additional search fees were accompanied by the applicant's protest but the applicable protest fee was not paid within the time limit specified in the invitation.

                                                ☐   No protest accompanied the payment of additional search fees.

Form PCT/ISA/210 (continuation of first sheet (2)) (January 2015)

INTERNATIONAL SEARCH REPORT
Information on patent family members

International application No.

**PCT/KR2020/003411**

| Patent document cited in search report | Publication date | Patent family member | Publication date |
|---|---|---|---|
| KR 10-1515394 B1 | 04/05/2015 | None | |
| KR 10-2014-0116374 A | 02/10/2014 | AU 2012-326203 A1 | 15/05/2014 |
| | | AU 2012-326203 A2 | 22/05/2014 |
| | | AU 2012-326203 B2 | 30/11/2017 |
| | | BR 112014009346 A2 | 18/04/2017 |
| | | CA 2852672 A1 | 25/04/2013 |
| | | CN 103987836 A | 13/08/2014 |
| | | CN 103987836 B | 10/04/2018 |
| | | CN 107058101 A | 18/08/2017 |
| | | DK 2768942 T3 | 27/01/2020 |
| | | EP 2768942 A1 | 27/08/2014 |
| | | EP 2768942 B1 | 04/12/2019 |
| | | EP 3608394 A1 | 12/02/2020 |
| | | HR P20200051 T1 | 20/03/2020 |
| | | JP 2014-533936 A | 18/12/2014 |
| | | JP 2018-023395 A | 15/02/2018 |
| | | JP 6219832 B2 | 25/10/2017 |
| | | JP 6629272 B2 | 15/01/2020 |
| | | KR 10-2058568 B1 | 22/01/2020 |
| | | PT 2768942 T | 21/01/2020 |
| | | RU 2014119926 A | 27/11/2015 |
| | | RU 2656156 C2 | 31/05/2018 |
| | | US 2014-0287509 A1 | 25/09/2014 |
| | | US 2019-0093073 A1 | 28/03/2019 |
| | | WO 2013-059343 A1 | 25/04/2013 |
| JP 2016-526923 A | 08/09/2016 | CN 105593366 A | 18/05/2016 |
| | | EP 3030652 A2 | 15/06/2016 |
| | | EP 3030652 A4 | 14/06/2017 |
| | | US 2015-0044750 A1 | 12/02/2015 |
| | | US 2015-0232800 A1 | 20/08/2015 |
| | | US 9029109 B2 | 12/05/2015 |
| | | WO 2015-021270 A2 | 12/02/2015 |
| | | WO 2015-021270 A3 | 02/04/2015 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140287509 A